# EUROPÄISCHE PATENTSCHRIFT

(11) **EP 0 914 073 B1**
(45) Veröffentlichungstag und Bekanntmachung des Hinweises auf die Patenterteilung: **13.02.2002**
(21) Anmeldenummer: 98929271.9
(22) Anmeldetag: 29.04.1998
(51) Int. Cl.: A61F 5/00

(54) **CERVIKALSTÜTZE**
CERVICAL BRACE
MINERVE

(30) Priorität: 02.05.1997 DE 19718674
(43) Veröffentlichungstag der Anmeldung: 12.05.1999
(73) Patentinhaber: Giebeler, Wolfgang, 61267 Neu-Anspach (DE)
(72) Erfinder: Giebeler, Wolfgang, 61267 Neu-Anspach (DE)
(74) Vertreter: Luderschmidt, Schüler & Partner GbR
(86) Internationale Anmeldenummer: EP9802521
(87) Internationale Veröffentlichungsnummer: WO9849978

(56) Entgegenhaltungen:
- DE-A1- 2 404 683
- US-A- 4 205 667
- US-A- 5 211 623

## Beschreibung

Die Erfindung betrifft eine Cervikalstütze nach dem Oberbegriff des Patentanspruchs 1.

Eine um den Hals des Patienten legbare Cervikalstütze ist aus der EP 0 385 114 B1 bekannt. Diese Cervikalstütze hat sich in der Praxis bewährt, nachteilig ist jedoch, daß die Cervikalstütze am Hals des Patienten verrutschen kann.

Eine andere Cervikalstütze der im Patentanspruch 1 angegebenen Art ist aus der US 5,211,623 bekannt. Die bekannte Cervikalstütze weist einen Stützkörper auf, der aus zwei rollenförmigen Segmenten besteht, die in einem schlauchförmigen Element übereinander angeordnet sind. Die rollenförmigen Segmente sind mit einem weichen Material gefüllt. Nachteilig ist, daß das untere Segment den Halsansatzbereich nicht eng umschließt und somit in diesem Bereich nur ein geringe Einschränkung der Bewegungsfreiheit gewährleistet ist.

Eine Cervitalstütze gemäß Oberbegriff des Anspruchs 1 ist aus DE-A-24 04 683 bekannt geworden.

Der Erfindung liegt die Aufgabe zugrunde, eine Cervikalstütze mit einer erhöhten Bewegungseinschränkung und gleichzeitig hohem Tragekomfort zu schaffen, bei der nicht die Gefahr des Verrutschens besteht.

Die Lösung der Aufgabe erfolgt erfindungsgemäß mit den Merkmalen des Patentanspruchs 1.

Der Stützkörper der erfindungsgemäßen Cervicalstütze wird von einem oberen und unteren Segment gebildet, die durch eine Schwächungszone voneinander getrennt sind. Das obere Segment sitzt im Halsbereich wie eine herkömmliche Cervikalstütze, während sich das untere Segment um den gesamten Halsansatzbereich legt. Das untere Segment, das den Halsansatzbereich eng umschließt, verhindert ein Verschieben der Cervikalstütze zirkulär um den Hals, so daß diese vom Patienten sogar in der Nacht getragen werden kann. Ohne den Tragekomfort zu beeinträchtigen, führt das untere Segment zu einer erhöhten Einschränkung des Bewegungsbereichs der Halswirbelsäule und hält die Muskulatur warm. Daraus ergibt sich eine bessere Muskelentspannung als bei einer herkömmlichen Cervikalstütze.

Der Stützkörper weist zumindest über einen Teil seiner Länge ein Profil auf, das sich zu dem unteren Rand hin verjüngt. Während der den Halsbereich umschließende Teil des Stützkörpers, d.h. das obere Segment, relativ formstabil ist, hat der den Halsansatz umschließende Teil des Stützkörpers, d.h. das untere Segment, eine größere Flexibilität, so daß dieses an dem Halsansatzbereich eng anliegt.

Im Sinne einer optimalen Paßform hat die Schwächungszone vorzugsweise einen Verlauf, der im wesentlichen der unteren Randkontur des Stützkörpers folgt.

Als Schwächungszone ist vorteilhafterweise eine Nut in dem Stützkörper vorgesehen. Aufgrund der geringen Materialstärke des Stützkörpers im Nutgrund wird eine ausreichende Flexibilität in dem Übergangsbereich zwischen dem oberen und unteren Segment erreicht. Anstelle einer Nut kann in den Stützkörper aber auch ein Materialstreifen eingelegt sein, der eine höhere Flexibilität aufweist.

In einer bevorzugten Ausführungsform ist die Nut an der Außenseite des Stützkörpers vorgesehen, wobei diese eine im wesentlichen rechtwinklig zu der Oberfläche des Stützkörpers verlaufende obere Flanke und eine dazu schräg verlaufende untere Flanke aufweist. Von Vorteil ist bei. dieser Ausführungsform, daß die obere Flanke der Nut einen Anschlag für das untere Segment des Stützkörpers bildet. Der Winkel zwischen den Flanken der Nut kann dabei so bemessen sein, daß der Stützkörper bei einem bestimmten Grad der Auslenkung des unteren Segments im Bereich der Schwächungszone wieder an Festigkeit gewinnt.

Der Stützkörper besteht vorzugsweise aus einem elastischen, aber dennoch ausreichend formstabilen Schaumstoffkörper, der ggf. mit einem Überzug aus hautverträglichem textilen Material versehen ist. Vorzugsweise wird der Stützkörper aus einem Stück rund geschäumt. Die Cervikalstütze kann aber auch als flacher Körper ausgebildet sein, der um den Hals des Patienten gelegt wird. Sie kann in unterschiedlichen Größen zur Verfügung gestellt werden, um eine optimale Paßform sicherzustellen.

Im folgenden wird ein Ausführungsbeispiel der Erfindung unter Bezugnahme auf die Zeichnungen näher erläutert.

Es zeigen:
- Figur 1: eine bevorzugte Ausführungsform der Cervikalstütze am Hals des Patienten in der Vorderansicht,
- Figur 2: die Cervikalstütze von Figur 1 in Seitenansicht,
- Figur 3: die Cervikalstütze von Figur 1 in der Rückansicht,
- Figur 4: der Stützkörper der flach ausgebreiteten Cervikalstütze von Figur 1 in der Draufsicht,
- Figur 5: der Stützkörper der flach ausgebreiteten Cervikalstütze von Figur 1 in der Unteransicht,
- Figur 6: eine Ansicht aus der Richtung des Pfeils VI von Fig. 4,
- Figur 7: einen Schnitt entlang der Linie VII - VII von Figur 4 und
- Figur 8: eine Schnitt entlang der Linie VIII - VIII von Figur 4.

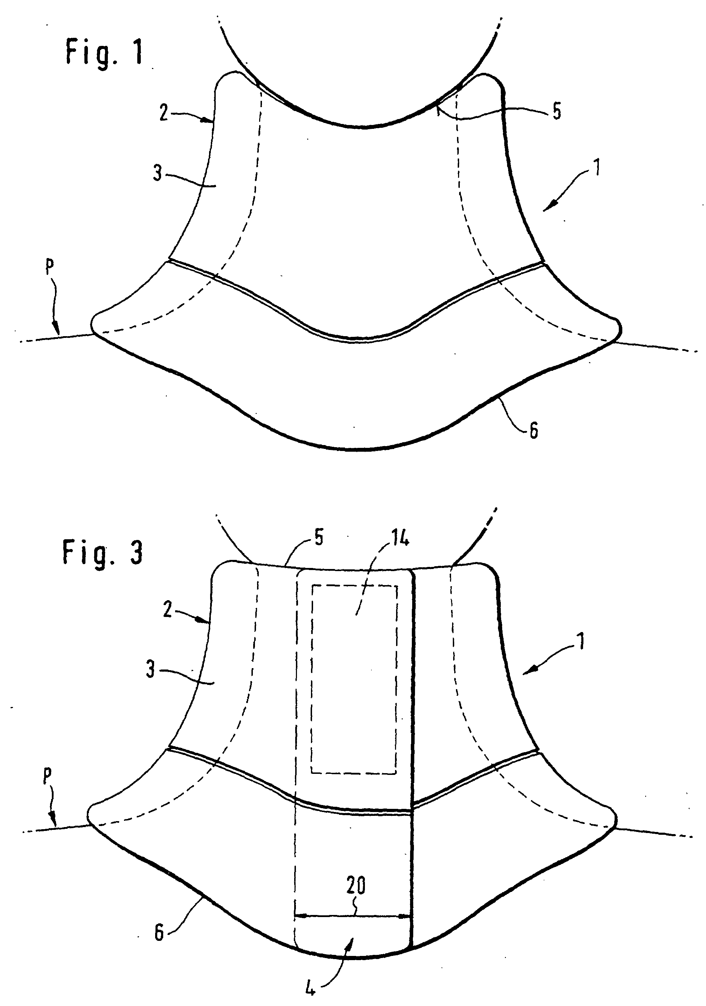

Die Figuren 1 bis 3 zeigen die Cervikalstütze 1 am Hals des Patienten P, die im wesentlichen aus einem flexiblen, aber ausreichend formstabilen Schaumstoffkörper 2 besteht, der mit einem hautverträglichen Überzug 3 aus textilem Material versehen ist. Der Schaumstoff mit einem Raumgewicht von vorzugsweise 40 kg/m² bildet einen Körper, der zum Anlegen und Abnehmen aufgrund seiner Flexibilität auseinandergespreitzt werden kann. Figur 4 zeigt den Stützkörper 2 der flach ausgebreiteten Cervikalstütze in der Draufsicht, d.h. die Außenseite des Stützkörpers, während die Innenseite des Stützkörpers in Figur 5 dargestellt ist.

Der Stützkörper ist an seinem oberen und unteren Umfangsrand 5, 6 der Anatomie des an den Hals angrenzenden Kopf- und Schulterbereichs des Patienten angepaßt. An seinem oberen Rand 5 ist der Stützkörper im Bereich der Kinnpartie 7 leicht ausgeschnitten. Der Ausschnitt 8 geht zu beiden Seiten in eine schrägverlaufende Abstützung 9 für den Kiefer über. Der Abstützung 9 folgt ein im wesentlichen gerader Abschnitt 10, an den sich ein zu den Enden hin leicht zurücklaufender Abschnitt 11 anschließt.

Der Stützkörper 2 ist an seinem unteren Rand 6 zu beiden Seiten des mittleren Abschnitts ausgeschnitten. Den Ausschnitten 12 folgen schräg nach unten verlaufende Abschnitte 13, die im wesentlichen zu den Enden des Stützkörpers hin gerade auslaufen und sich am Hals des Patienten im Nackenrückenbereich teilweise überlappen. Zum Verschließen sind an den Enden des Stützkörpers Klettverschlüsse 14 vorgesehen, die auf den Überzug 3 aufgenäht sind (Figur 3).

Der Stützkörper 2 weist ein oberes und ein unteres Segment 15, 16 auf. Das obere Segment 15 wird von dem unteren Segment 16 durch eine längslaufende Nut 17 an der Außenseite des Stützkörpers getrennt. Die Nut 17 hat einen Verlauf, der mit Ausnahme des Nackenrückenbereichs 14 der Kontur des unteren Randes 15 des Stützkörpers folgt. Im Nackenrückenbereich 14 läuft die Nut 17 zu beiden Seiten des Stützkörpers hin im wesentlichen gerade aus. Die Nut 17 weist eine rechtwinklig zur Oberfläche des Stützkörpers 2 verlaufende obere Flanke 18 und eine schräg dazu verlaufende untere Flanke 19 auf. Die Tiefe der Nut 17 ist derart, daß der Stützkörper 2 im Nutgrund über die gesamte Länge im wesentlichen die gleiche Materialstärke hat. Das untere Segment 16 hat eine Breite; die etwa 20 bis 40%, vorzugsweise 30% der Breite des gesamten Stützkörpers entspricht.

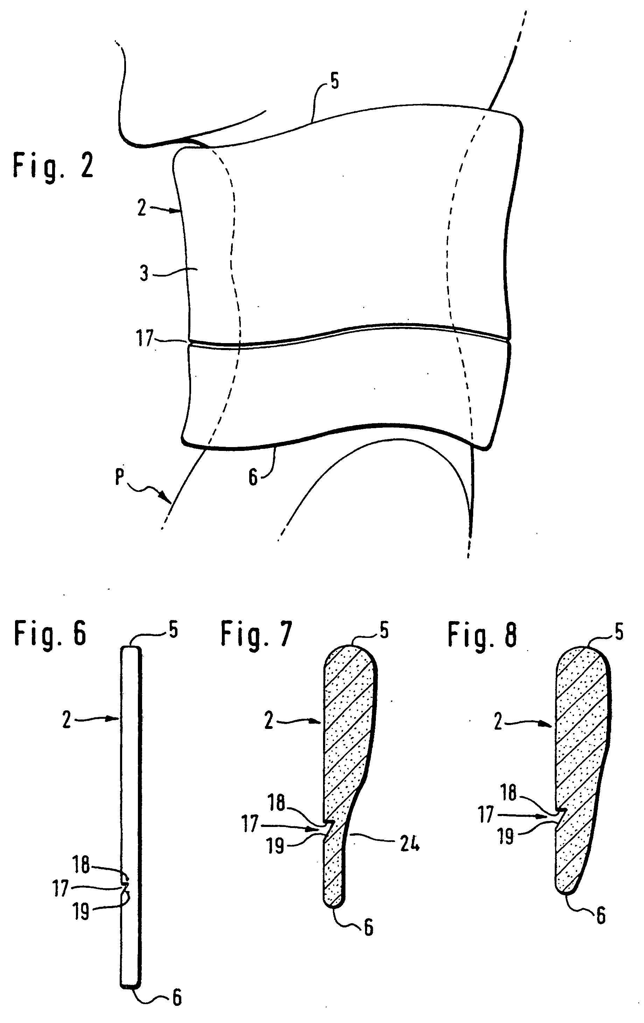

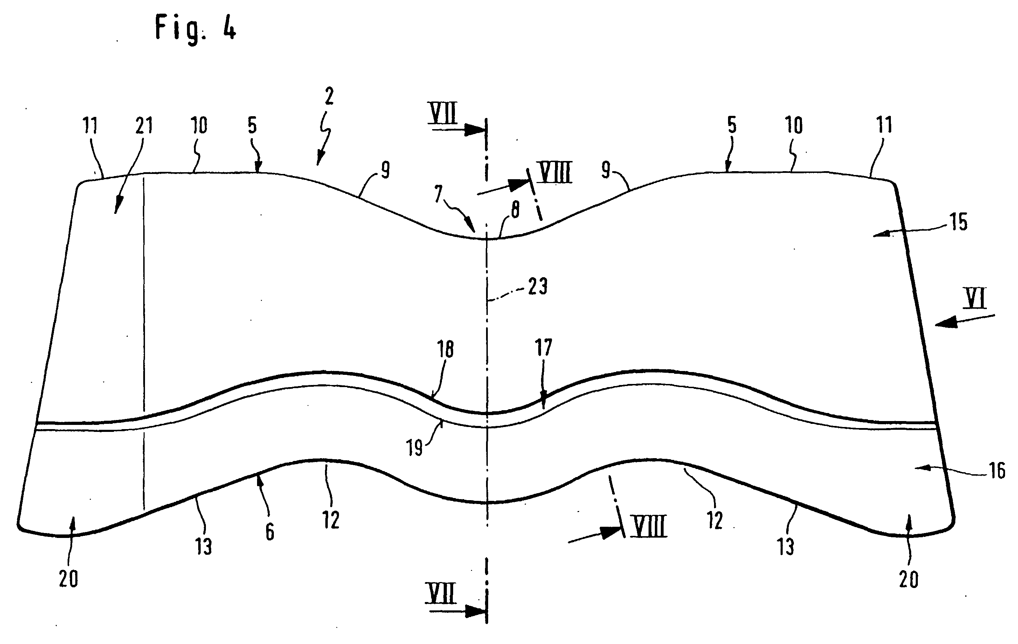

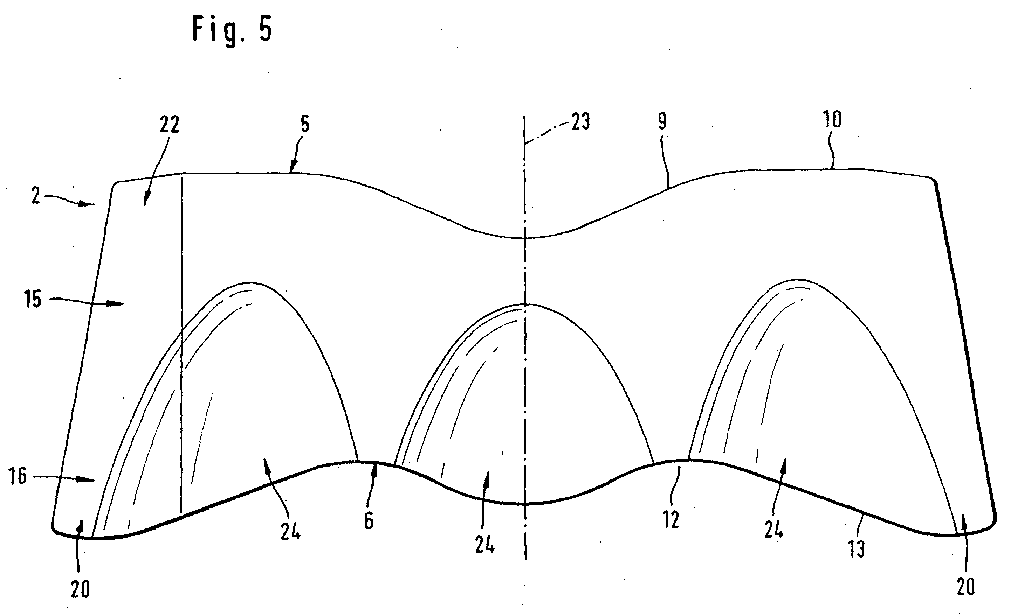

Der Stützkörper 2 weist im Überlappungsbereich 20 an der Außenseite des einen und der Innenseite des anderen Endes eine Abschrägung 21, 22 auf. Ansonsten ist die Profilierung des Stützkörpers bezüglich der mit dem Bezugszeichen 23 versehenen Mittellinie symmetrisch, so daß nachfolgend nur die Profilierung der in Figur 4 gezeigten rechten Seite anhand der Figuren 5-7 beschrieben wird. Die Außenseite des Stützkörpers 2 mit Ausnahme des Überlappungsbereichs 20 ist flach. Die Innenseite ist hingegen derart profiliert, daß der Stützkörper eng im Halsbereich und unteren Halsansatzbereich des Patienten anliegt und diesen Bereich fest umschließt (Figuren 1-3). Im Bereich der Kinnpartie 7 weist der Stützkörper die größte Materialstärke auf, während sich das Profil zum unteren Randbereich hin verjüngt. Zu beiden Seiten des Kinnbereichs 7 schließen sich flache muldenförmige Vertiefungen 24 an, die sich bis zu dem Überlappungsbereich 20 erstrecken und die derart ausgebildet sind, daß der Stützkörper zu dem unteren Rand hin abgeschrägt ist (Figuren 6 und 7).

Entlang der unteren Randkontur 6 und entlang der oberen Randkontur 5, abgesehen vom Überlappungsbereich 20, hat der Stützkörper im wesentlichen die gleiche Materialstärke, wobei die Materialstärke am unteren Rand etwa 20 bis 40%, vorzugsweise 30%, der Materialstärke am oberen Rand entspricht.

Das sich im Halsansatzbereich abstützende untere Segment verhindert, daß sich die Cervicalstütze zirkulär um den Hals verschiebt und erhöht die Bewegungseinschränkung der Halswirbelsäule, woraus eine erhöhte Muskelentspannung folgt, ohne daß der Tragekomfort beeinträchtigt wird. Dieser Effekt wird noch durch den anatomischen Innenzuschliff des Stützkörpers verstärkt.

## Patentansprüche

1. Cervikalstütze mit einem um den Hals des Patienten legbaren und an seinen Schmalseiten lösbar miteinander verbindbaren Stützkörper (2), dessen obere und untere Randkonturen (5, 6) an die Anatomie des an den Hals angrenzenden Kopf- und Schulterbereichs angepaßt sind, **dadurch gekennzeichnet daß** der Stützkörper aus einem oberen und unteren Segment gebildet wird, die durch eine Schwächungszone (17) voneinander getrennt sind, und daß der Stützkörper (2) zumindest über einen Teil seiner Länge ein sich zu dem unteren Rand hin verjüngendes Profil aufweist, so daß das untere Segment (16) beim Anlegen der Cervikalstütze den Halsansatzbereich eng umschließen kann.

2. Cervikalstütze nach Anspruch 1, **dadurch gekennzeichnet, daß** die Schwächungszone (17) einen im wesentlichen der unteren Randkontur (5) des Stützkörpers (2) folgenden Verlauf hat.

3. Cervikalstütze nach Anspruch 2, **dadurch gekennzeichnet, daß** als Schwächungszone in dem Stützkörper (2) eine Nut (17) vorgesehen ist.

4. Cervikalstütze nach Anspruch 3, **dadurch gekennzeichnet, daß** die Nut (17) an der Außenseite des Stützkörpers (2) vorgesehen ist, wobei diese eine im wesentlichen rechtwinklig zu der Oberfläche des Stützkörpers verlaufende obere Flanke (18) und eine dazu schräg verlaufende untere Flanke (19) aufweist.

5. Cervicalstütze nach einem der Ansprüche 1 bis 4, **dadurch gekennzeichnet, daß** der Stützkörper (2) entlang der unteren Randkontur (6) im wesentlichen die gleiche Materialstärke hat.

6. Cervicalstütze nach einem der Ansprüche 1 bis 5, **dadurch gekennzeichnet, daß** der Stützkörper ein elastischer Schaumstoffkörper (2) ist.

7. Cervicalstütze nach Anspruch 6, **dadurch gekennzeichnet, daß** der elastische Schaumstoffkörper (2) mit einem Überzug (3) aus textilem Material versehen ist.

## Claims

1. Cervical brace with a brace body (2) that can be placed around the neck of the patient and which can be detachably connected at its narrow ends, the upper and lower edge contours (5, 6) of which body are adapted to the anatomy of the head and shoulder area adjacent to the neck, **characterised in that** the brace body is formed from an upper and lower segment which are separated from each other by an attenuation area (17) and **in that** the brace body (2) exhibits a profile which is reduced towards the lower edge, at least over part of its length, so that the lower segment (16) is able to enclose the neck base area when the cervical brace is placed round the neck.

2. Cervical brace according to Claim 1, **characterised in that** the attenuation area (2) is developed essentially following the lower edge contour (5) of the brace body (2).

3. Cervical brace according to Claim 2, **characterised in that** a groove (17) is provided as the attenuation area in the brace body (2).

4. Cervical brace according to Claim 3, **characterised in that** the groove (17) is provided on the outside of the brace body (2), this groove exhibiting an upper flank (18) running essentially at right angles to the surface of the brace body and a lower flank (19) running obliquely to the upper flank.

5. Cervical brace according to one of Claims 1 to 4, **characterised in that** the brace body (2) has essentially the material thickness along the lower edge contour (6).

6. Cervical brace according to one of Claims 1 to 5, **characterised in that** the brace body is an elastic foam body (2).

7. Cervical brace according to Claim 6, **characterised in that** the elastic foam body (2) is provided with a cover (3) of textile material.

## Revendications

1. Minerve comprenant un corps de support (2) apte à venir se placer autour du cou du patient et dont les petits côtés peuvent être reliés l'un à l'autre de manière amovible, dont les contours marginaux supérieur et inférieur (5, 6) épousent la forme de l'anatomie des zones de la tête et des épaules limitrophes au cou, **caractérisée en ce que** le corps de support est constitué par un segment supérieur et par un segment inférieur qui sont séparés l'un de l'autre par une zone d'affaiblissement (17) et **en ce que** le corps de support (2) présente, au moins sur une partie de sa longueur, un profil qui se rétrécit en direction du bord inférieur de telle sorte que le segment inférieur (16) lors du placement de la minerve est en mesure d'entourer étroitement la zone de la base du cou.

2. Minerve selon la revendication 1, **caractérisée en ce que** la zone d'affaiblissement (17) possède une allure qui suit essentiellement le contour marginal inférieur (5) du corps de support (2).

3. Minerve selon la revendication 2, **caractérisée en ce qu'**on prévoit, à titre de zone d'affaiblissement dans le corps de support (2), une rainure (17).

4. Minerve selon la revendication 3, **caractérisée en ce qu'**on prévoit la rainure (17) sur le côté externe du corps de support (2), ce côté présentant un flanc supérieur (18) s'étendant essentiellement à angle droit par rapport à la surface du corps de support et un flanc inférieur (19) s'étendant en inclinaison par rapport au premier cité.

5. Minerve selon l'une quelconque des revendications 1 à 4, **caractérisée en ce que** le corps de support (2) possède, le long du contour marginal inférieur (6), essentiellement la même épaisseur de matière.

6. Minerve selon l'une quelconque des revendications 1 à 5, **caractérisée en ce que** le corps de support est un corps élastique en mousse (2).

7. Minerve selon la revendication 6, **caractérisée en ce que** le corps élastique en mousse (2) est muni d'un revêtement (3) constitué d'une matière textile.
